# EUROPÄISCHE PATENTSCHRIFT

(11) **EP 2 887 971 B1**
(45) Veröffentlichungstag und Bekanntmachung des Hinweises auf die Patenterteilung: **28.06.2017**
(21) Anmeldenummer: 13740321.8
(22) Anmeldetag: 26.07.2013
(51) Int. Cl.: A61L 2/26, B65D 90/00

(54) **STERILISIERBEHÄLTER MIT ZENTRIERHILFE FÜR BEHÄLTERDECKEL**
STERILISATION CONTAINER WITH CENTRING AID FOR CONTAINER LID
RECIPIENT DE STERILISATION AVEC SYSTÈME D'AIDE AU CENTRAGE POUR COUVERCLES DE CONTENANTS

(30) Priorität: 24.08.2012 DE 102012215121
(43) Veröffentlichungstag der Anmeldung: 01.07.2015
(73) Patentinhaber: Aesculap AG, 78532 Tuttlingen (DE)
(72) Erfinder: WEIßHAUPT, Dieter, 78194 Immendingen (DE); SCHUSTER, Stefan, 79865 Grafenhausen (DE); SCHWEIZER, Matthias, 78532 Tuttlingen (DE); GRAY-DREIZLER, John, 78628 Rottweil (DE); ZIERIS, Gerold, 78570 Mühlheim (DE)
(74) Vertreter: Koller, Tobias Kilian
(86) Internationale Anmeldenummer: PCT/EP2013/065851
(87) Internationale Veröffentlichungsnummer: WO 2014/029587

(56) Entgegenhaltungen:
- EP-A1- 2 404 567
- EP-A1- 2 478 921
- DE-A1- 10 230 519
- DE-U1-202012 100 997
- US-B1- 7 021 485

## Beschreibung

Die vorliegende Erfindung betrifft einen Behälter mit einer Behälterwanne mit einer Zugangsöffnung, einem Behälterdeckel. DE202012100997U offenbart einen Sterilisierbehälter, welcher ein Behälterunterteil und ein Behälteroberteil zum Verschließen des Behälterunterteils umfasst, welche Dichtung in sich geschlossen ausgebildet und umlaufend am Behälteroberteil angeordnet ist, einen Dichtelementhaltekörper zum Festlegen am Behälteroberteil und zwei am Dichtelementhaltekörper angeordnete, umlaufende Dichtlippen, umfasst, dadurch gekennzeichnet, dass mindestens eine der Dichtlippen, gelenkig am Dichtelementhaltekörper gehalten oder gelagert ist. Insbesondere bei Sterilisiercontainern, aber auch bei manch anderem Behälter, ist eine Dichtung zwischen dem Behälterdeckel und der Behälterwanne vorgesehen. Die Dichtung zwischen Behälterdeckel und Behälterwanne kann zum Beispiel eine Doppeldichtung sein, die in Form einer Klappdichtung ausgebildet ist. Bei diesem Dichtungstyp sind zwei Abdichtbereiche vorgesehen. Ein erster Abdichtungsabschnitt wird erzeugt, indem eine erste Dichtlippe der an dem Behälterdeckel montierten Dichtung auf die Stirnseite der Wandung der Behälterwanne aufgesetzt wird. Dabei wird die erste Dichtlippe gegenüber ihrem Befestigungsabschnitt nach oben verformt, wodurch eine zweite Dichtlippe, die unterhalb der ersten Dichtlippe angeordnet ist, nach innen verschoben wird, sodass sie sich seitlich an die Außenseite der Wandung der Behälterwanne anlegt und einen zweiten Abdichtungsabschnitt bildet. Je nach Dimensionierung der Dichtlippen, des Behälterdeckels und der Behälterwanne ist ein gewisses seitliches Spiel vorgesehen, mit dem der Behälterdeckel auf die Behälterwanne aufgesetzt werden kann. Seitliches Spiel meint hier ein Spiel in jeder Richtung innerhalb der Dichtebene. Die Dichtebene ist definiert durch den Verlauf der Dichtung entlang der Zugangsöffnung der Behälterwanne. Verläuft die Dichtung nicht in einer Ebene, ist die Dichtebene eine Normalebene auf den Vektor der Zugangsöffnung der Behälterwanne.

Wird der Behälterdeckel aber schräg oder versetzt auf die Behälterwanne aufgesetzt, so kann es passieren, dass auf einer Seite des Behälters die zweite Dichtlippe teilweise auf die Stirnseite der Wandung der Behälterwanne aufgesetzt wird, sodass vorstehendes Funktionsprinzip nicht mehr wirkt. Zugleich kann es passieren, dass auf der gegenüber liegenden Behälterseite die erste Dichtlippe zumindest teilweise neben der Wandung der Behälterwanne positioniert wird, sodass an dieser Stelle überhaupt kein Abdichtungsabschnitt zwischen Behälterdeckel und Behälterwanne ausgebildet wird und Luft ungehindert durch den entstandenen Spalt in und aus dem Behälter strömen kann.

Ähnliches kann auch passieren, wenn eine einfache Dichtung zwischen Behälterdeckel und Behälterwanne angeordnet wird. Als Material für solch eine Dichtung wird oft Silikon verwendet, da es gute elastische Eigenschaften hat und unter anderem auch einen Sterilisiervorgang unbeschadet übersteht. Da Silikondichtungen aber meistens sehr weich sind, geben sie dem Nutzer des Behälters kein ausgeprägtes haptisches Feedback darüber, ob der Behälterdeckel korrekt auf der Behälterwanne positioniert ist. Zudem eignet sich die Dichtung selbst aufgrund ihrer Weichheit wenig dafür, den Behälterdeckel zur Behälterwanne korrekt auszurichten. Die Dichtung kann aber auch aus einem anderen Elastomer hergestellt sein.

Es ist daher eine Aufgabe der vorliegenden Erfindung, eine Zentrierhilfe bzw. eine Positionierhilfe für einen Behälter bereit zu stellen, welche sicher stellt, dass der Behälterdeckel in einer korrekten Lage bzw. Position auf die Behälterwanne aufgesetzt wird und dadurch die Abdichtung des Behälterinneren gegenüber der Umgebung sicher gestellt wird. Abdichtung bedeutet hierbei nicht nur eine totale Abdichtung, die keinerlei Medienaustausch zwischen dem Behälterinneren und der Umgebung ermöglicht, sondern zum Beispiel auch eine Abdichtung, bei der sicher gestellt ist, dass ein Medienaustausch nur durch eine am Behälter vorgesehene Membran stattfindet, sodass kein unsteriles Medium in das Behälterinnere eintreten kann, wie es bei medizinischen Sterilisiercontainern öfter der Fall ist.

Die Aufgabe der vorliegenden Erfindung wird gelöst durch einen Sterilisierbehälter gemäß Anspruch 1. Weitere vorteilhafte Ausgestaltungen sind Gegenstand der abhängigen Ansprüche.

Gemäß einem Aspekt ist eine Zentrierhilfe bereit gestellt für einen Behälter mit einer Behälterwanne mit einer Zugangsöffnung, einem Behälterdeckel und einer Dichtung, welche im geschlossenen Zustand des Behälters zwischen der Behälterwanne und dem Behälterdeckel angeordnet ist. Die Zentrierhilfe weist eine erste Zentriereinrichtung, die an entweder dem Behälterdeckel oder der Behälterwanne vorgesehen ist, und eine zweite Zentriereinrichtung auf, die an dem anderen von entweder dem Behälterdeckel oder der Behälterwanne vorgesehen ist. Die erste und zweite Zentriereinrichtung wirken dabei derart zusammen, dass sicher gestellt ist, dass sich die Dichtung im geschlossenen Zustand des Behälters entlang des gesamten Umfangs der Zugangsöffnung mit der Behälterwanne und dem Behälterdeckel in Kontakt befindet.

Die Zugangsöffnung ist dabei die Öffnung, durch die Gegenstände in den Behälter eingebracht oder aus ihm heraus genommen werden können und befindet sich bei einem Behälter üblicherweise an der Oberseite der Behälterwanne. Die Dichtung ist üblicherweise ringförmig ausgebildet und entweder an der Behälterwanne oder dem Behälterdeckel angebracht. Bei medizinischen Sterilisiercontainern kommt es vor, dass die Dichtung lösbar an dem Containerdeckel angebracht ist. Es ist auch möglich, dass die Dichtung weder an der Behälterwanne noch an dem Behälterdeckel angebracht ist, jedoch ist die Handhabung des Behälters beim Öffnen und Schließen deutlich schneller und einfacher, wenn die Dichtung nicht lose zwischen den beiden Behälterteilen angeordnet ist. Häufiger jedoch wird die Dichtung an einem der Containerbauteile angeklebt, bevorzugt in den Containerdeckel eingeklebt, oder sie wird direkt an eines der Containerbauteile angespritzt. All dies kann selbstverständlich auch bei anderen Behältern als medizinischen Sterilisiercontainern so umgesetzt werden.

Die Aufgabe der Dichtung ist es, zu verhindern, dass ein Medium - üblicherweise Luft - zwischen dem Rand der Behälterwanne und dem Behälterdeckel strömen kann. Die Dichtung soll das Behälterinnere sicher von der Umgebung des Behälters trennen. Bei medizinischen Sterilisiercontainern ist dies der Fall, um einen Eintritt von unsteriler Luft oder anderen unsterilen Medien in das Containerinnere zu verhindern und somit die in dem Container befindlichen Instrumente und anderen Gegenstände sicher steril zu halten. Ein steriler Medienaustausch und Druckausgleich kann in diesem Fall beispielsweise über Filtermembrane oder andere Filtereinrichtungen gewährleistet sein.

Im geschlossenen Zustand des Behälters liegt der Behälterdeckel auf der Dichtung auf, welche wiederum auf der Behälterwanne aufliegt. Üblicherweise liegt die Dichtung dabei auf der Stirnseite der Wandung der Behälterwanne auf. Bei einer solchen Anordnung der Dichtung zwischen Behälterwanne und Behälterdeckel gilt es vorwiegend, eine horizontale Verschiebung bzw. einen horizontalen Versatz des Behälterdeckels zur Dichtung und/oder der Dichtung zur Behälterwanne zu vermeiden. Wenn die Dichtung an entweder dem Behälterdeckel oder der Behälterwanne angebracht ist, so muss nur der horizontale Versatz zwischen der Dichtung und dem jeweils anderen Behälterteil sicher gestellt werden. Ein Abheben des Behälterdeckels von der Dichtung und/oder der Dichtung von der Behälterwanne kann vermieden werden, indem der Behälterdeckel zu der Behälterwanne hin fixiert ist bzw. vorgespannt ist, beispielsweise durch einen Verschluss mit Verschlusslaschen.

Mit einem solchen Aufbau ist sicher gestellt, dass die Dichtung ihre Abdichtfunktion zwischen Behälterdeckel und Behälterwanne erfüllt, da sich die beiden Bauteile nicht so weit in horizontaler Richtung gegeneinander verschieben können, dass ein Spalt zwischen der Dichtung und einem der Behälterteile entsteht.

Gemäß einer vorteilhaften Ausgestaltung der vorliegenden Erfindung ist bei der Zentrierhilfe im geschlossenen Zustand des Behälters das Spiel zwischen der ersten und der zweiten Zentriereinrichtung geringer als das Spiel zwischen Behälterdeckel und Behälterwanne. Hier ist das Spiel zwischen Behälterdeckel und Behälterwanne in dem Fall gemeint, in dem keine Zentrierhilfe vorgesehen ist. Bei einem Behälter mit Zentrierhilfe begrenzt die Zentrierhilfe aufgabengemäß das Spiel zwischen Behälterdeckel und Behälterwanne, was nur funktioniert, wenn das Spiel zwischen der ersten und der zweiten Zentrierhilfe geringer ist als zwischen einem entsprechenden Behälterdeckel und einer Behälterwanne ohne Zentrierhilfe.

Das Spiel zwischen Behälterdeckel und Behälterwanne hängt stark von dem Material und der Form der Dichtung sowie von der Geometrie des Behälterdeckels und des Randes der Behälterwanne ab, auf die der Behälterdeckel aufgesetzt wird. Die Dichtung ist zumeist aus einem sehr elastischen und flexiblen Material hergestellt und weist eine gewisse Breite auf, damit das Aufsetzen des Behälterdeckels auf die Behälterwanne schnell, einfach und ohne allzu große Aufmerksamkeit des Nutzers erfolgen kann. Die Dichtung wird bei einem schrägen Aufsetzen des Behälterdeckels auf die Behälterwanne verformt und trägt nicht zu einer Zentrierung des Behälterdeckels zu der Behälterwanne bei. Auch die Geometrie des Behälterdeckels und der Behälterwanne sollen ein schnelles, einfaches und sicheres Verschließen des Behälters ermöglichen.

Gemäß einer weiteren vorteilhaften Ausgestaltung der vorliegenden Erfindung weist die erste Zentriereinrichtung eine Vielzahl von männlichen Zentrierelementen auf und die zweite Zentriereinrichtung weist eine Vielzahl von weiblichen Zentrierelementen auf. Das Zentrieren des Behälterdeckels zu der Behälterwanne erfolgt dabei dadurch, dass die männlichen Zentrierelemente in die weiblichen Zentrierelemente eindringen und von diesen aufgenommen werden, oder aber dadurch, dass sich die männlichen Zentrierelemente an den weiblichen Zentrierelementen in Anlage geraten. Dabei erstrecken sich die männlichen Zentrierelemente zumindest anteilig zu den weiblichen Zentrierelementen der jeweils anderen Zentriereinrichtung hin.

Gemäß noch einer weiteren vorteilhaften Ausgestaltung der vorliegenden Erfindung weisen die erste und zweite Zentriereinrichtung je wenigstens ein männliches und ein weibliches Zentrierelement auf. Mit einer solchen Ausgestaltung können an Behälterdeckel und Behälterwanne je männliche und weibliche Zentrierelemente vorgesehen sein.

Gemäß einer anderen vorteilhaften Ausgestaltung der vorliegenden Erfindung weist wenigstens ein männliches Zentrierelement im Wesentlichen die Form eines Parallelepipeds - insbesondere eines Quaders - , einer Pyramide, eines Pyramidenstumpfes, eines Prismas, eines Zylinders, eines Kegels, eines Kegelstumpfes oder eines Teilellipsoids auf. Oder aber das wenigstens eine männliche Zentrierelement ist aus mehreren gleichen und/oder verschiedenen der vorstehend genannten Körper zusammen gesetzt. Das zugehörige weibliche Zentrierelement weist vorzugsweise eine korrespondierende negative Form auf, sodass das männliche Zentrierelement im Wesentlichen formschlüssig in dem weiblichen Zentrierelement aufgenommen ist. Alternativ dazu kann das weibliche Zentrierelement aber auch eine andere Form aufweisen und ein Formschluss und damit die Zentrierung bzw. Positionierung der beiden Zentriereinrichtungen zueinander wird nicht über die gesamte axiale Länge des männlichen und weiblichen Zentrierelementes hergestellt sondern nur über gewisse Abschnitte derselben. Ein Beispiel hierfür ist ein kegelförmiges männliches Zentrierelement, welches in einem zylindrischen weiblichen Zentrierelement aufgenommen ist, wobei die Radien der kreisförmigen Grundflächen beider Zentrierelemente im Wesentlichen identisch sind. Bei einer solchen Konstellation tritt ein Formschluss zwischen den beiden Zentrierelementen in Querrichtung zur Achse der Zentrierelemente nur im Bereich des Fußes des kegelförmigen männlichen Zentrierelements auf. Ansonsten ist zwischen den beiden Zentrierelementen ein Spalt bzw. ein Hohlraum ausgebildet.

Gemäß einer weiteren vorteilhaften Ausgestaltung der vorliegenden Erfindung weist wenigstens ein männliches Zentrierelement und/oder ein weibliches Zentrierelement einen zumindest teilweise konischen Bereich auf, sodass das Spiel zwischen männlichem und weiblichem Zentrierelement zu Beginn des Aufsetzens des Behälterdeckels größer ist als im geschlossenen Zustand des Behälters. Dabei weist das zugehörige weibliche Zentrierelement und/oder männliche Zentrierelement vorzugsweise eine korrespondierende negative bzw. positive Form auf. Mit einem solchen Aufbau muss der Behälterdeckel beim Aufsetzen zunächst nur recht grob zu der Behälterwanne zentriert werden, um einen ersten - zunächst recht losen - Eingriff der männlichen und zugehörigen weiblichen Zentrierelemente zu erzielen. Wird der Behälterdeckel nun weiter auf die Behälterwanne aufgesetzt, nähert sich der Querschnitt der männlichen und weiblichen Zentrierelemente in dem konischen Bereich entweder schrittweise oder kontinuierlich aneinander an. Die Form der beiden Zentrierelemente korrespondiert deshalb vorteilhafter Weise, damit sich kein Schmutz in dem ansonsten geschaffenen Hohlraum sammeln kann. Die Ausbildung von Hohlräumen zwischen den Zentrierelementen könnte bei medizinischen Sterilisiercontainern dazu führen, dass sich Schmutz in den Hohlräumen sammelt und möglicherweise beim Öffnen in den Container fällt und die dort befindlichen Instrumente und Gegenstände zumindest scheinbar kontaminiert. Der Schmutz selbst wäre zwar mit sterilisiert worden, aber das ist dem Schmutz nicht anzusehen. Zudem wird der Schmutz vermutlich erst bemerkt, wenn er in der Behälterwanne liegt, was den Eindruck erweckt, dass die Instrumente und Geräte vor dem Sterilisieren nicht ordnungsgemäß gereinigt wurden. Diesem Problem kann man abhelfen, indem man entweder keine Hohlräume zwischen den Zentrierelementen schafft oder aber solche Hohlräume über eine Spülöffnung mit der Umgebung verbindet, sodass eventuell vorhandener Schmutz ausgespült werden kann. Für den Fall, dass kein Hohlraum zwischen den männlichen und weiblichen Zentrierelementen geschaffen wird, verhindert Schmutz, der sich in einem weiblichen Zentrierelement gesammelt hat, dass das männliche Zentrierelement vollständig in dem weiblichen Zentrierelement aufgenommen wird und somit auch, dass der Behälterdeckel vollständig auf die Behälterwanne aufgesetzt werde kann und der Container verschlossen werden kann. Dies zeigt dem Nutzer, dass eine Verschmutzung zumindest eines Zentrierelements vorliegen muss, sodass er die Möglichkeit hat, diese Verschmutzung vor dem Verschließen des Behälters zu entfernen. Auf diese Weise wird eine Verschmutzung bei einem medizinischen Sterilisiercontainer vor der Reinigung und Sterilisation des Containers entdeckt und beseitigt, wodurch es nicht passieren kann, dass eine Verschmutzung beim Öffnen des Containers zutage treten kann und möglicherweise in den Container fallen kann.

Gemäß noch einer anderen vorteilhaften Ausgestaltung der vorliegenden Erfindung sind die Zentriereinrichtungen so angeordnet, dass sie im geschlossenen Zustand des Behälters außerhalb des Behälterinneren angeordnet sind. Auf diese Weise wird der nutzbare Raum im Behälterinneren durch die Zentriereinrichtung nicht verkleinert.

Zudem kann auf diese Weise besser visuell kontrolliert werden, ob der Behälterdeckel korrekt auf die Behälterwanne aufgesetzt ist, sogar noch im vollständig geschlossenen Zustand.

Gemäß noch einer vorteilhaften Ausgestaltung der vorliegenden Erfindung ist die erste Zentriereinrichtung direkt oder über je eine an den äußeren Stirnseiten der Behälterwanne angeordnete Frontblende an den äußeren Stirnseiten vorgesehen, und/oder die zweite Zentriereinrichtung ist in der Umgebung von Verschlusselementen vorgesehen, welche an mindestens zwei entgegen gesetzten Seiten des Behälterdeckels vorgesehen sind. Mit der ersten beschriebenen Ausbildung muss die erste Zentriereinrichtung nicht direkt an oder mit der Behälterwanne ausgebildet werden, sondern sie kann an oder mit der Frontblende oder Teilen von dieser ausgebildet werden und anschließend an der Behälterwanne montiert werden. Es kann auch zwischen einer Stirnseite des Behälters und eine Frontblende auch noch ein weiteres Bauteil angeordnet sein, an dem die erste Zentriereinrichtung vorgesehen ist. Dieses Bauteil kann beispielsweise eine Grundplatte sein, die fest mit der Stirnseite verbunden ist und eine Montagefläche für die Frontblende bereit stellt. Auf diese Weise werden eine leichtere Montage und eine größere Materialauswahl für die erste Zentriereinrichtung gewährleistet. Die beiden Stirnseiten sind relativ weit voneinander (in Bezug auf die Abmessungen des Behälters), sodass sich die Zentriereinrichtung insgesamt über einen großen Bereich des Behälters erstreckt und somit aufgrund des wenn auch geringen Spiels zwischen den Zentriereinrichtungen eine geringere Verdrehung des Behälterdeckels zu der Behälterwanne zulässt, als wenn die Zentriereinrichtung nur eine recht kleine Abmessung hätte. Mit der zweiten beschriebenen Ausbildung ist die zweite Zentriereinrichtung in der Umgebung von Verschlusselementen vorgesehen, mit deren Hilfe der Behälterdeckel an der Behälterwanne gehalten wird oder der Behälterdeckel zu der Behälterwanne hin gespannt wird. Der Nutzer schenkt dem Bereich der Verschlusselemente beim Schließvorgang erhöhte Aufmerksamkeit, da dies der Verschluss erfordert. Von daher kann er die korrekte Zentrierung des Behälterdeckels zu der Behälterwanne besonders einfach und leicht überprüfen, wenn sich die zweite Zentriereinrichtung in dem Bereich der Verschlusselemente befindet. Außerdem lässt die zweite Ausbildung auch zu, dass ein Behälterdeckel mit mehr als zwei Verschlusselementen an der Behälterwanne befestigt wird und die Zentrierelemente dementsprechend verteilt sind. Beispielsweise kann ein quadratischer Behälter mit je einem Verschlusselement an jeder Seite des Behälterdeckels vorgesehen sein. Beiderseits jedes Verschlusselements ist je ein männliches und/oder weibliches Zentrierelement vorgesehen und die Behälterwanne verfügt über korrespondierende Verschlusselementaufnahmen und weibliche und/oder männliche Zentrierelemente.

Gemäß einer besonderen Ausbildung kann die Zentrierhilfe auch Teil des Verschlusses sein. Beispielsweise kann an einem Verschlusselement, welches schwenkbar am Behälterdeckel vorgesehen ist, ein Zentrierelement vorgesehen sein. Es ist aber auch möglich, dass das schwenkbare Verschlusselement selbst ein Zentrierelement darstellt. In diesem Fall wird der Behälterdeckel zunächst auf die Behälterwanne aufgesetzt. Anschließend wird das Verschlusselement zu der Behälterwanne hin geschwenkt. Dabei gelangen die Seitenflächen des Verschlusselements, welches hierbei das männliche Zentrierelement darstellt, in Kontakt mit Anlageflächen an der Verschlusselementaufnahme, welche das weibliche Zentrierelement darstellen. Bei dem Schwenkvorgang wird der Kontakt der Seitenflächen zwischen Verschlusselement und Verschlusselementaufnahme zunächst in dem Bereich hergestellt, der der Schwenkachse des Verschlusselements am nächsten ist und pflanzt sich dann zum freien Ende des Verschlusselements hin fort. Sind die Seitenflächen von Verschlusselement und Verschlusselementaufnahme derart schräg zu der Achse des Verschlusselements angeordnet, dass sie sich zum freien Ende des Verschlusselements aneinander annähern, so wird auch auf diese weise eine Selbstzentrierung des Behälterdeckels zu der Behälterwanne erzielt. Durch spezielle Ausgestaltung der Dicke des Verschlusselements oder durch Vorsehen einer Biegung entlang der Länge des Verschlusselements kann der Vorgang der Selbstzentrierung eingestellt werden.

Gemäß einer weiteren vorteilhaften Ausgestaltung der vorliegenden Erfindung weist die erste Zentriereinrichtung vier flächige Vorsprünge auf, welche in lateraler Richtung einen Winkel gegenüber der Behälterlängsachse und der Behälterquerachse einnehmen, wobei die Vorsprünge jeweils beiderseits einer Verschlussaufnahme an der Oberseite von zwei Frontblenden vorgesehen sind, die an beiden äußeren Stirnseiten der Behälterwanne angeordnet sind. Die flächigen Vorsprünge sind dabei an ihrer lateralen Stirnseite stärker abgeschrägt als an ihrer zentralen Stirnseite, sodass sich die Vorsprünge in distaler Richtung im Wesentlichen von außen her verjüngen. Die zweite Zentriereinrichtung weist zudem vier Vertiefungen auf, die daran angepasst sind, die entsprechenden flächigen Vorsprünge im Wesentlichen formschlüssig aufzunehmen und jeweils zwei Vertiefungen sind in einem Bauteil ausgebildet, welches zwischen der inneren Stirnseite des Behälterdeckes und der Dichtung vorgesehen ist. Diese Ausbildung

Gemäß noch einer anderen vorteilhaften Ausgestaltung der vorliegenden Erfindung ist die erste Zentriereinrichtung an der Innenseite des Behälterdeckels innerhalb der Dichtung vorgesehen und die zweite Zentriereinrichtung ist an der Behälterwanne vorgesehen. Mit dieser Ausbildung wird eine Zentrierhilfe geschaffen, die im Inneren des Behälters angeordnet ist. Die erste Zentriereinrichtung kann dann beispielsweise aus mindestens zwei männlichen Zentrierelementen bestehen, die an mindestens zwei verschiedenen Seiten des Behälterdeckels innerhalb der Dichtung aber in der Nähe des Behälterrands angeordnet sind und sich zumindest anteilig nach unten erstrecken. Die weiblichen Zentriereinrichtungen bestehen dabei aus schrägen Anlageflächen, die an der Innenseite der Wandung der Behälterwanne im Bereich des oberen Rands der Behälterwanne ausgebildet sind. Die schrägen Anlageflächen sind derart gestaltet, dass sie bei einer Betrachtung von oben nach unten von außen nach innen verlaufen. Kommen die männlichen Zentrierelemente in Kontakt mit einer oder mehreren der weiblichen Zentrierelemente, sorgt dieser Kontakt für eine Selbstzentrierung im weiteren Verlauf des Schließvorgangs, bis alle männlichen Zentrierelemente an den zugehörigen weiblichen Zentrierelementen anliegen und der Behälter geschlossen ist.

Gemäß einer weiteren vorteilhaften Ausgestaltung der vorliegenden Erfindung besteht die erste Zentriereinrichtung aus einer geraden Anzahl von männlichen Zentrierelementen, die in den Ecken des Behälterdeckels und/oder in der Mitte jeder Seite des Behälterdeckels angeordnet sind. Besonders vorteilhaft ist hierbei die Anordnung in den Ecken, da die Zentrierelemente auf diese Weise maximal beabstandet sind und das vorhandene geringe Spiel zwischen männlichen und weiblichen Zentrierelementen dadurch den geringsten Einfluss auf die Lage des Behälterdeckels zu der Behälterwanne hat.

Gemäß einer weiteren vorteilhaften Ausgestaltung der vorliegenden Erfindung sind die Zentriereinrichtungen aus einem Material ausgebildet, das einen höheren Elastizitätsmodul als die Dichtung aufweist, vorzugsweise aus einem steifen Kunststoff oder Metall. Auf diese Weise kann man mit einfachen geometrischen Formen der männlichen und weiblichen Zentrierelemente eine gute Zentrierung sicher stellen. Als Metall kommt besonders Aluminium und Legierungen mit Aluminium in Betracht und als Kunststoff beispielsweise PEEK. Für die Dichtung wird häufig Silikon verwendet, was einen sehr niedrigen Elastizitätsmodul aufweist, sodass es zahlreiche mögliche Materialien für die Zentrierelemente gibt. Es ist aber auch möglich, die männlichen Zentrierelemente aus einem härteren Material auszubilden als die weiblichen Zentrierelemente und die beiden Materialien danach auszusuchen, dass eine besonders geringe Reibung zwischen den männlichen und weiblichen Zentrierelementen entsteht. Dies erhöht den Komfort bei der Benutzung des Behälters. Die männlichen Zentrierelemente sind eher einer Stoßbelastung oder einer anderen unbeabsichtigten Belastung ausgesetzt als die weiblichen Zentrierelemente, was es nahe legt, diese aus dem stabileren Material auszubilden. Dies schließt aber nicht aus, dass die männlichen Zentrierelemente aus dem weicheren Material ausgebildet werden, um die Gefahr zu verringern, dass durch die Vorsprünge, die die männlichen Zentrierelemente bilden, Verletzungen verursacht werden, indem diese beispielsweise an Sollbruchstellen versagen. In diesem Fall können die männlichen und/oder weiblichen Zentrierelemente auswechselbar vorgesehen werden.

Gemäß einer weiteren vorteilhaften Ausgestaltung der vorliegenden Erfindung hat die Zentrierhilfe einen punktsymmetrischen Aufbau mit einem Drehwinkel von 360°/n, wobei n eine positive ganze Zahl ist, vorzugsweise eine ganze Zahl größer eins. Mit dieser Ausgestaltung kann das erfindungsgemäße Prinzip auch bequem auf Behälter angewendet werden, die beispielsweise eine dreieckige, quadratische oder sechseckige (hexagonalen) Form haben. Aber auch für einen gewöhnlichen rechteckigen Behälter hat dieser Aufbau den Vorteil, dass es nicht nur eine Lage bzw. Ausrichtung des Behälterdeckels zu der Behälterwanne gibt, in der die Zentriereinrichtungen ineinander eingreifen können, sondern der Behälterdeckel kann um 360°/n gedreht werden und passt trotzdem auf die Behälterwanne. Es gibt also für jeden Behälterdeckel n korrekte Lagen bzw. Ausrichtungen relativ zu der Behälterwanne und nicht nur eine einzige. Man könnte dementsprechend sagen, dass der Behälterdeckel eines rechtwinkligen Behälters auf diese Weise kein vorn und hinten kennt sondern in beiden Ausrichtungen auf die Behälterwanne passt.

Weitere Vorteile und Merkmale der Erfindung sind dem Fachmann aus den beigefügten Figuren und der detaillierten Beschreibung der Ausführungsbeispiele ersichtlich.
- Fig. 1: zeigt eine isometrische Ansicht eines medizinischen Sterilisiercontainers mit einer Zentrierhilfe gemäß dem ersten Ausführungsbeispiel;
- Fig. 2: zeigt eine isometrische Ansicht einer Containerwanne des medizinischen Sterilisiercontainers aus Fig. 1;
- Fig. 3: zeigt eine isometrische Ansicht eines Containerdeckels des medizinischen Sterilisiercontainers aus Fig. 1;
- Fig. 4: zeigt eine perspektivische Ansicht eines Ausschnitts der Fig. 3;
- Fig. 5: zeigt eine weitere perspektivische Ansicht eines Ausschnitts der Fig. 3;
- Fig. 6: zeigt eine perspektivische Ansicht eines Bauteils mit zwei weiblichen Zentrierelementen gemäß einem ersten Ausführungsbeispiel;
- Fig. 7: zeigt eine Ansicht einer Containerwanne des medizinischen Sterilisiercontainers aus Fig. 2 von vorn;
- Fig. 8: zeigt eine Ansicht eines Ausschnitts einer Containerwanne des medizinischen Sterilisiercontainers aus Fig. 2 von oben;
- Fig. 9: zeigt eine perspektivische Ansicht eines Ausschnitts einer Containerwanne des medizinischen Sterilisiercontainers aus Fig. 2;
- Fig. 10: zeigt eine perspektivische Ansicht eines Bauteils mit zwei männlichen Zentrierelementen gemäß einem ersten Ausführungsbeispiel;
- Fig. 11: zeigt einen horizontalen Schnitt durch den Containerdeckel aus Fig. 3;
- Fig. 12: zeigt einen vertikalen Schnitt durch den Containerdeckel und die Containerwanne entsprechend Fig. 1;
- Fig. 13: zeigt einen weiteren vertikalen Schnitt durch den Containerdeckel und die Containerwanne entsprechend Fig. 1;
- Fig. 14: zeigt eine schematische Ansicht eines männlichen und eines weiblichen Zentrierelements gemäß einem zweiten Ausführungsbeispiel;
- Fig. 15: zeigt eine schematische Ansicht eines männlichen und eines weiblichen Zentrierelements gemäß einem dritten Ausführungsbeispiel;
- Fig. 16: zeigt eine schematische Ansicht eines männlichen und eines weiblichen Zentrierelements gemäß einem vierten Ausführungsbeispiel; und
- Fig. 17: zeigt eine schematische Ansicht eines männlichen und eines weiblichen Zentrierelements gemäß einem fünften Ausführungsbeispiel.

Im Folgenden sind die Ausführungsbeispiele unter Bezugnahme auf die Figuren im Detail beschrieben.

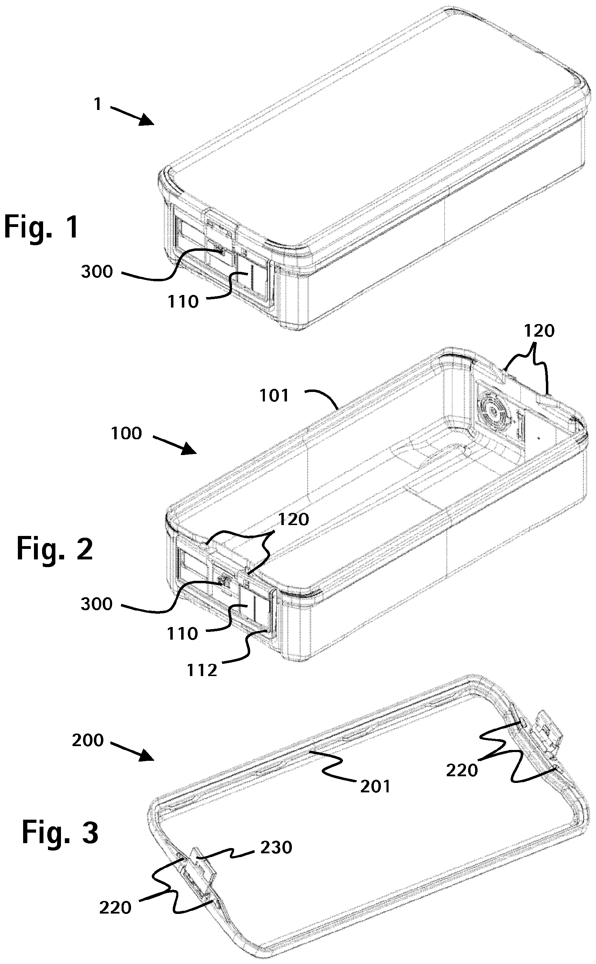

Ein erstes Ausführungsbeispiel der vorliegenden Erfindung ist im Folgenden unter Bezugnahme auf die Fig. 1 bis 13 im Detail beschrieben.

Wie dies in der Fig. 1 gezeigt ist, ist der Behälter 1 des ersten Ausführungsbeispiels ein medizinischer Sterilisationscontainer 1 mit einer Containerwanne 100 und einem Containerdeckel 200. Die Containerwanne ist mit einer Frontblende 110, einer Sterilanzeige 111 und einem Griff 112 versehen. Eine Frontblende 110 ist an beiden Stirnseiten der Containerwanne 100 vorgesehen, wobei sich ihr Aufbau teilweise unterscheiden kann. Der Containerdeckel 200 verfügt über eine Dichtung 201, die entlang des Randes des Containerdeckels 200 verläuft, mit dem Containerdeckel 200 verklebt ist und mit dem Rand 101 der Containerwanne 100 zusammenwirkt, um den Innenraum des Containers 1 sicher zu verschließen. Der Containerdeckel 200 verfügt über zwei Verschlusslaschen 230, die schwenkbar sind und jeweils in eine entsprechende Verschlussaufnahme 130 an der äußeren Stirnseite der Containerwanne 100 eingeführt und dort verriegelt werden können, um einen Verschluss zwischen Containerdeckel 200 und Containerwanne 100 sicher zu stellen. Eine Sicherheitsplombe 300 kann durch die Verschlusslasche 230 gesteckt und in der Verschlussaufnahme 130 verankert werden, um dem Nutzer sicher anzuzeigen, dass der Container 1 nach einem Sterilisationsvorgang noch nicht geöffnet wurde.

Eine erste Zentriereinrichtung bestehend aus männlichen Zentrierelementen 120 ist an der Containerwanne 100 vorgesehen und eine zweite Zentriereinrichtung bestehend aus weiblichen Zentrierelementen 220 ist an der Containerwanne vorgesehen. Die erste und zweite Zentriereinrichtung sind daran angepasst, so zusammenwirken, dass sicher gestellt ist, dass sich die Dichtung 201 im geschlossenen Zustand des Containers entlang des gesamten Umfangs der Zugangsöffnung zu dem Inneren des Containers 1 mit der Containerwanne 100, genauer gesagt mit dem Rand 101 der Containerwanne 100, und dem Containerdeckel 200 in Kontakt befindet. In diesem Fall wirken die erste und zweite Zentriereinrichtung derart zusammen, dass die weiblichen Zentrierelemente 220 auf die männlichen Zentrierelemente 120 geschoben werden. Indem zwischen den männlichen und weiblichen Zentrierelementen 120, 220 im geschlossenen Zustand des Containers 1 nur ein gewisses Spiel vorgesehen ist, welches geringer ist als die Toleranz bzw. das Spiel der Dichtung 201 gegenüber einer schiefen Positionierung des Deckels 200 gegenüber der Wanne 100, wird auf diese Weise sicher gestellt, dass die Dichtung 201 den Container 1 sicher dicht verschießt. Die Dichtung 201 ist dabei so gestaltet und angeordnet, dass sie, wenn keine Zentriereinrichtung an dem Container 1 vorgesehen ist, ein größeres Spiel bei der Positionierung von Deckel 200 zu Wanne 100 erlaubt, ohne das die Dichtwirkung der Dichtung 201 verloren geht. Im vorliegenden Fall ist die Dichtung 201 eine Doppeldichtung mit zwei Dichtlippen. Eine Dichtlippe liegt von oben auf dem Rand 101 der Containerwanne 100 auf, wodurch eine zweite Dichtlippe von außen gegen die Außenwand der Containerwanne 100 gedrängt wird. Dies geschieht, indem die erste Dichtlippe nach oben bewegt wird, was zu einer Verdrehung der zweiten Dichtlippe nach innen führt. Eine solche Dichtung weist eine recht hohe Toleranz gegenüber einem schrägen Aufsetzen des Deckels 200 auf die Wanne 100 auf. Selbstverständlich ist die vorliegende Erfindung auch auf andere Behälter und Container anwendbar, die mit anderen Dichtungen versehen sind, beispielsweise mit Dichtungen mit nur einer Dichtlippe.

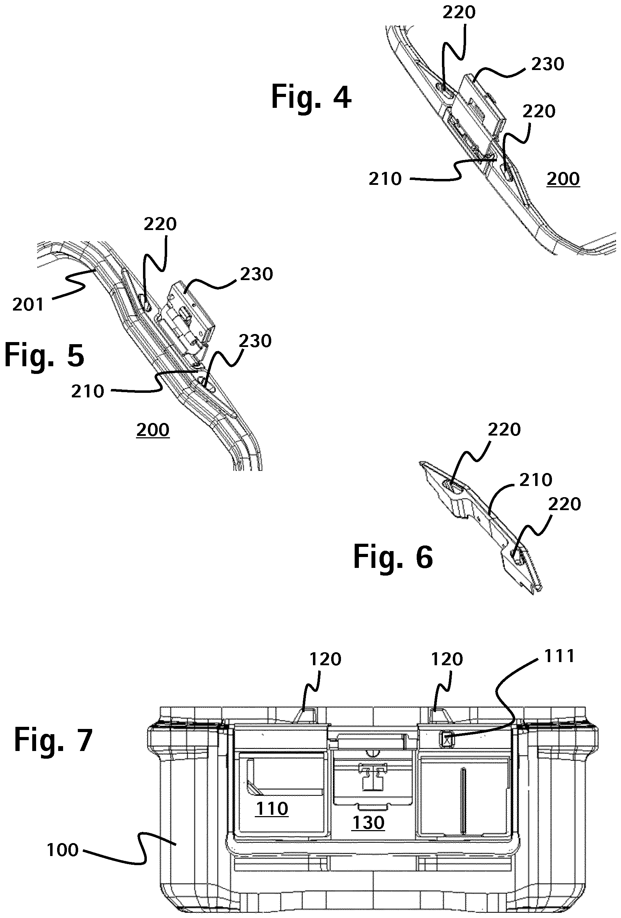

Die männlichen Zentrierelemente 120 gemäß diesem Ausführungsbeispiel bestehen aus einem im Wesentlichen flächigen Körper. Bei einer Ansicht des Containers 1 von vorn entsprechend der Fig. 7 weisen die männlichen Zentrierelemente 120 eine steile Innenwand und eine demgegenüber flachere Außenwand auf, sodass sie eine trapezähnliche Form bilden. Die beiden Seitenwände der männlichen Zentrierelemente sind im Wesentlichen parallel zueinander. Die weiblichen Zentrierelemente 220 gemäß diesem Ausführungsbeispiel haben eine Form, die der Form der männlichen Zentrierelemente 120 entspricht und lediglich ein bisschen größer sind, sodass die männlichen Zentrierelemente 120 ohne Zwang in den weiblichen Zentrierelementen 220 aufgenommen werden können.

Durch die relativ flach ansteigende Außenwand und die relativ steil ansteigende Innenwand der männlichen Zentrierelemente 120 sind diese konisch ausgebildet. Auf diese Weise ist das Spiel zwischen einem männlichen und einem weiblichen Zentrierelement zu Beginn des Aufsetzens des Behälterdeckels größer ist als im geschlossenen Zustand des Behälters. Die männlichen Zentrierelemente 120 und die weiblichen Zentrierelemente 220 sind jeweils außerhalb des Containerinneren angeordnet. Die männlichen Zentrierelemente 120 sind dabei an Frontblenden 110 angeordnet, welche an den äußeren Stirnseiten der Containerwanne 100 angebracht sind. Die weiblichen Zentrierelemente 220 sind jeweils paarweise an einem Bauteil 210 vorgesehen, welches im Bereich der Verschlusslaschen 230 zwischen der Dichtung 201 und den Verschlusslaschen 230 an dem Containerdeckel 200 angebracht ist, also an den Stirnseiten des Containerdeckels 200. Der obere Rand 101 der Containerwanne 100 bestimmt bei dem Container 1 die Zugangsöffnung und die Dichtung 201 ist an den Verlauf des oberen Rands 101 der Containerwanne 100 angepasst.

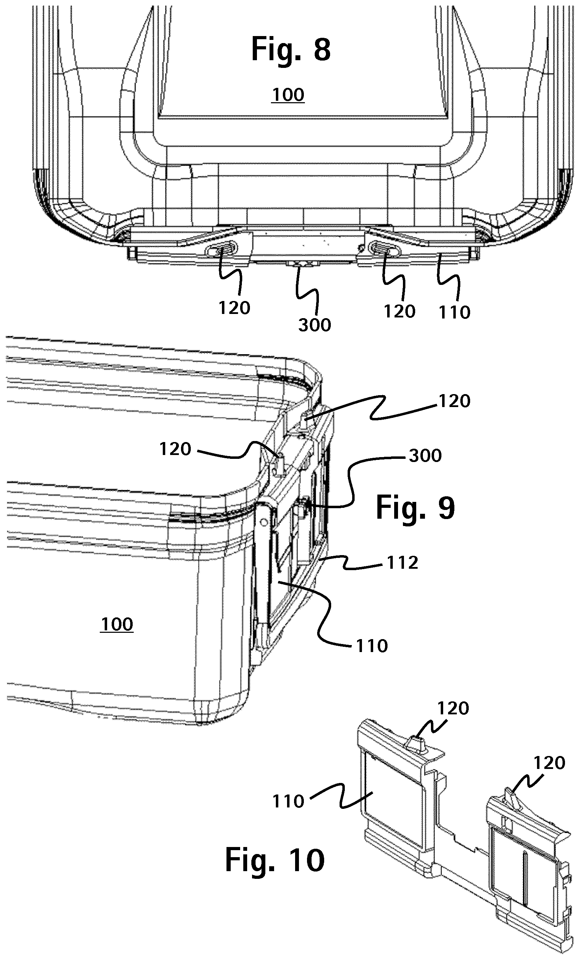

Die erste Zentriereinrichtung dieses Ausführungsbeispiels weist demnach vier flächige Vorsprünge 120 auf, nämlich die vier männlichen Zentrierelemente 120. Diese nehmen, wie dies besonders gut in Fig.8 und 9 gezeigt ist, in lateraler Richtung einen Winkel gegenüber der Containerlängsachse und der Containerquerachse ein, wobei die männlichen Zentrierelemente 120 jeweils beiderseits einer Verschlussaufnahme 130 an der Oberseite von zwei Frontblenden 110 vorgesehen sind, die an beiden äußeren Stirnseiten der Containerwanne 100 angeordnet sind. Die männlichen Zentrierelemente 120 sind an ihrer lateralen Stirnseite stärker abgeschrägt sind als an ihrer zentralen Stirnseite, sodass sich die Vorsprünge in distaler Richtung im Wesentlichen von außen her verjüngen, wie dies in den Fig. 2, 7, 8 und 12 gut zu erkennen ist.

Bei dem vorliegenden Ausführungsbeispiel sind die männlichen Zentrierelemente aus demselben Material wie die Frontblenden 110 ausgebildet, nämlich aus Edelstahl. Die weiblichen Zentrierelemente 220 sind in dem Bauteil 210 ausgebildet, welches bei diesem Ausführungsbeispiel aus Kunststoff, genau genommen aus PEEK (Polyetheretherketon), hergestellt ist. Auf diese Weise sind die Zentriereinrichtungen aus einem Material ausgebildet, das einen höheren Elastizitätsmodul als die Dichtung aufweist, die in diesem Fall aus Silikon hergestellt ist.

Die Zentrierhilfe gemäß diesem Ausführungsbeispiel bestehend aus je vier männlichen und weiblichen Zentrierelementen 120, 220 weist einen punktsymmetrischen Aufbau mit einem Drehwinkel von 180° auf. Dies bedeutet, dass der Containerdeckel 200 in zwei verschiedenen, um 180° gedrehten Lagen auf die Containerwanne 100 aufgesetzt werden kann.

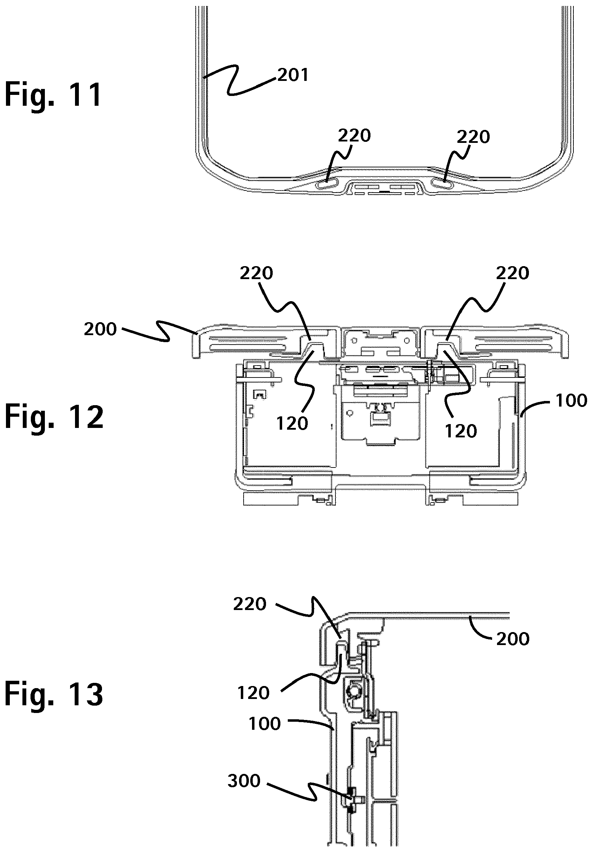

In den Fig. 12 und 13 ist gezeigt, wie die männlichen Zentrierelemente 120 in den weiblichen Zentrierelementen 220 aufgenommen sind.

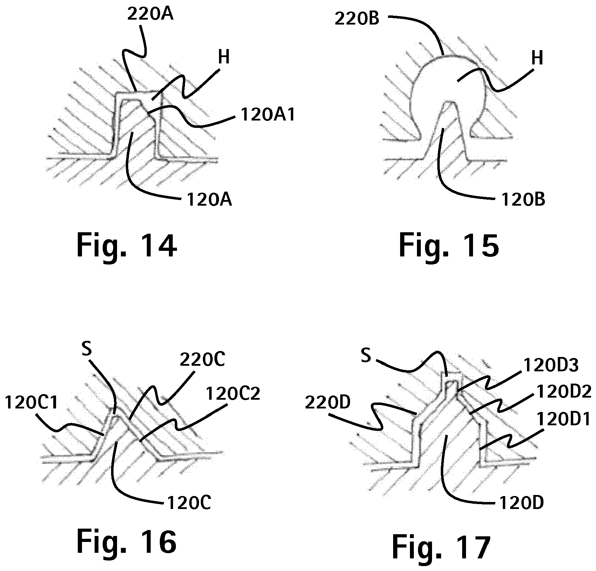

Im Folgenden ist ein zweites Ausführungsbeispiel der vorliegenden Erfindung unter Bezugnahme auf die Fig. 14 beschrieben. Im Anschluss wird nur auf die Unterschiede zum ersten Ausführungsbeispiel eingegangen, da der überwiegende Teil der Merkmale beider Ausführungsbeispiele identisch ist.

Die männlichen Zentrierelemente 120A dieses Ausführungsbeispiels bestehen aus einem Quader, von dem ein dreiseitiges Prisma entfernt wurde. Auf diese Weise entsteht eine schräge Fläche 120A1, die einen konischen Teil am männlichen Zentrierelement 120A ausbildet und dabei hilft, den Containerdeckel 200 zur Containerwanne 100 auszurichten. Die weiblichen Zentrierelemente 220A haben die Form einer quaderförmigen Aussparung 220A. Zwischen einem männlichen Zentrierelement 120A und einem weiblichen Zentrierelement 220A wird somit ein Hohlraum H ausgebildet. Die Form der männlichen und weiblichen Zentrierelemente 120A, 220A muss also nicht korrespondierend ausgebildet sein.

Außerdem sind bei diesem Ausführungsbeispiel die männlichen Zentrierelemente 120A am Containerdeckel 200 vorgesehen und die weiblichen Zentrierelemente 220A sind an der Containerwanne 100 vorgesehen. Dabei können die Zentrierelemente 120A, 220A jeweils direkt oder indirekt über weitere Bauteile an der Containerwanne 100 und dem Containerdeckel 200 vorgesehen sein. Bei diesem Ausführungsbeispiel ist der Container zudem quadratisch. An dem Containerdeckel 200 sind zudem vier Verriegelungslaschen 230 vorgesehen, jeweils eine mittig an jeder Seite des Containerdeckels 200. Die Containerwanne 100 verfügt dementsprechend über vier Verriegelungsaufnahmen 110 mittig an jeder Seite der Containerwanne 1. Die Vorderseite und die Rückseite des Containers 1 ist jeweils mit einer Frontblende 110 versehen in die jeweils eine Verriegelungsaufnahme 110 ausgebildet ist. Die Verriegelungsaufnahmen 110, welche an den beiden lateralen Wänden des Containers 1 vorgesehen sind, sind an zusätzlichen Blenden ausgebildet, die von außen an den Seitenwänden der Containerwanne 100 angebracht sind. Je zwei weibliche Zentrierelemente 220A sind an jeder Frontblende 110 und jeder Seitenblende ausgebildet. Der Containerdeckel 200 ist bei diesem Ausführungsbeispiel mit vier Bauteilen 210 versehen, wobei in diesen Bauteilen 210 nun die männlichen Zentrierelemente 120A ausgebildet sind. Auf diese Weise erhält man einen Containerdeckel 200, der in vier verschiedenen Stellungen auf die Containerwanne 100 aufsetzbar ist, nämlich jeweils um 90° in der Waagrechten gedreht. Um zu verhindern, dass sich in dem Hohlraum H Verschmutzungen sammeln, kann der Hohlraum H über einen Kanal mit der Umgebung verbunden sein, sodass bei einem Reinigungsvorgang eventuell in dem Hohlraum H befindliche Verschmutzungen mit dem Spülfluid ausgespült werden. Alternativ dazu können die weiblichen Zentrierelemente 220A auch an ihrer den männlichen Zentrierelementen 110A abgewandten Seite vollständig offen sein, also hier als rechteckige Durchgangslöcher ausgebildet sein.

Im Folgenden ist ein drittes Ausführungsbeispiel der vorliegenden Erfindung unter Bezugnahme auf die Fig. 15 im Detail beschrieben, wobei hier wieder nur die Unterschiede zum ersten Ausführungsbeispiel beschrieben sind.

Bei diesem Ausführungsbeispiel werden die männlichen Zentrierelemente 120B durch einen leicht abgestumpften Kegel gebildet. Demnach sind die männlichen Zentrierelemente 120B bei diesem Ausführungsbeispiel rotationssymmetrisch ausgebildet. Die weiblichen Zentrierelemente 220B haben in etwa die Form eines kugelförmigen Hohlraumes, welcher zu den männlichen Zentrierelementen 120B hin offen ist. Auch bei diesen Zentrierelementen 120B, 220B wird ein Hohlraum H ausgebildet, der alternativ auch wieder durch einen Kanal mit der Umgebung verbunden sein kann. Zudem sind bei diesem Ausführungsbeispiel jeweils männliche Zentrierelemente 120B und weibliche Zentrierelemente 220B in Umfangsrichtung der Containerwanne 100 und des Containerdeckels 200 abwechselnd an diesen vorgesehen, wobei sich immer ein männliches Zentrierelement 120B und ein weibliches Zentrierelement 220B gegenüber stehen. Durch diese Anordnung der männliche und weiblichen Zentrierelemente120B, 220B bleibt der punktsymmetrische Aufbau von Containerwanne 100 und Containerdeckel 200 erhalten, d.h. der Containerdeckel 200 kann noch immer in zwei um 180° gedrehten Stellungen auf die Containerwanne 100 aufgesetzt werden.

Ein viertes Ausführungsbeispiel der vorliegenden Erfindung ist im Folgenden unter Bezugnahme auf die Fig. 16 im Detail beschrieben, wobei hier wiederum nur dessen Unterschiede zum ersten Ausführungsbeispiel beschrieben sind. Die männlichen Zentrierelemente 120C dieses Ausführungsbeispiels sind als vierseitige schräge Pyramiden ausgebildet. Die Fig. 17 zeigt einen Schnitt durch ein männliches und weibliches Zentrierelement 120C, 220C in beiden Hauptrichtungen. Dies bedeutet, das männliche Zentrierelement 120C weist zwei steile Flanken 120C1 und zwei flache Flanken 120C2 auf. Die beiden steilen Flanken 120C1 sind jeweils zum Zentrum des Containers 1 hin angeordnet und die beiden flachen Flanken 120C2 sind jeweils vom Zentrum des Containers 1 weg angeordnet. Die weiblichen Zentrierelemente 220C haben eine den männlichen Zentrierelementen 120C entsprechende Form. Auch in diesem Fall kann ein Kanal vorgesehen sein, der den Spalt S, der zwischen männlichen und weiblichen Zentrierelementen 120C, 220C gebildet wird, mit der Umgebung verbindet. Auf diese Weise kann sicher gestellt werden, dass sich keine Verschmutzung in den weiblichen Zentrierelementen 120C fest setzt und so verhindert, dass die männlichen Zentrierelemente 120C korrekt in die weiblichen Zentrierelemente 220C eingeführt werden können. Bevorzugt wird solch ein Kanal an oder im Bereich des Bodens (der tiefste Teil) der weiblichen Zentrierelemente ausgebildet.

Selbstverständlich ist es bei diesem wie auch bei anderen Ausführungsbeispielen möglich, die steilen Flanken der männlichen und weiblichen Zentrierelemente nach außen hin anzuordnen. Bevorzugt sollte lediglich darauf geachtet werden, dass die Zentrierelemente punktsymmetrisch vorgesehen werden.

Im Folgenden ist ein fünftes Ausführungsbeispiel der vorliegenden Erfindung unter Bezugnahme auf die Fig. 17 beschrieben. Im Anschluss wird wieder nur auf die Unterschiede zum ersten Ausführungsbeispiel eingegangen, da der überwiegende Teil der Merkmale beider Ausführungsbeispiele wieder identisch ist.

Die männlichen Zentrierelemente 120D dieses Ausführungsbeispiels sind aus drei Abschnitten aufgebaut, einem zylindrischen Sockel 120D1, einem kegelstumpfförmigen Mittelteil 120D2 und einer wiederum zylindrischen Spitze 120D3. Die weiblichen Zentrierelemente 220D haben eine entsprechende negative Form, wobei wiederum ein schmaler Spalt S zwischen den beiden Zentrierelementen 120D, 220D ausgebildet ist. Solche Zentrierelemente 120D, 220D sind vorteilhaft, da während des Aufsetzens des Containerdeckels 200 auf die Containerwanne 100 zunächst ein relativ großes Spiel zwischen Containerdeckel 200 und Containerwanne 100 vorhanden sein kann, da zunächst nur die Spitze 120D3 des männlichen Zentrierelements 120D in das weibliche Zentrierelement 220D eingeführt werden muss. Bei fortschreitendem Aufsetzen des Containerdeckels 200 auf die Containerwanne 100 kommt die geneigte Oberfläche des Mittelteils 120D2 in Anlage an die Begrenzung des weiblichen Zentrierelements 220D und sorgt für eine Selbstzentrierung des Containerdeckels 200 zur Containerwanne 100, indem die Begrenzung des weiblichen Zentrierelements 220D an der schrägen Fläche des Mittelteils 120D2 des männlichen Zentrierelements 120D entlang gleitet, bis auch der Sockel 120D1 in das weibliche Zentrierelement 220D eingeführt werden kann.

Darüber hinaus sollten die männlichen und weiblichen Zentrierelemente so gestaltet und dimensioniert sein, dass die Verschlusselemente 210 nur dann in die Verriegelungsaufnahmen 110 eingeführt werden können, wenn der Containerdeckel vollständig zur Containerwanne zentriert ist, d.h. die männlichen Zentrierelemente vollständig in den weiblichen Zentrierelementen aufgenommen sind.

Gemäß einem weiteren Ausführungsbeispiel sind die Zentrierelemente innerhalb des Containerinneren vorgesehen. So sind in den Eckbereichen der Containerwanne Zentrierflächen vorgesehen, die vom oberen Rand 101 der Containerwanne 100 zu deren Boden hin nach innen zulaufen. Diese Zentrierflächen entsprechen dann weiblichen Zentrierelementen, an denen beispielsweise stabförmige Zentrierelemente, welche innerhalb der Dichtung 201 an der Innenseite des Containerdeckels 200 in den Eckbereichen des Containerdeckels 200 vorgesehen sind, entlang gleiten, während der Containerdeckel 200 auf die Containerwanne 100 aufgesetzt wird. Die stabförmigen Zentrierelemente entsprechen dann den männlichen Zentrierelementen.

Die an der Containerwanne vorgesehenen Zentrierelemente können bei allen Ausführungsbeispielen direkt mit der Containerwanne ausgebildet sein oder sie können an Frontblenden oder anderen blenden angeordnet sein oder an weiteren Bauteilen, die an der Containerwanne vorgesehen sind. Die Zentrierelemente müssen auch nicht paarweise angeordnet sein. Es kann auch eine ungerade Anzahl von Zentrierelementen einer Sorte vorgesehen sein, wobei dann entsprechend viele Zentrierelemente der anderen Sorte vorgesehen sind. Es können auch insgesamt mehr weibliche als männliche Zentrierelemente vorgesehen sein, sodass einige weibliche Zentrierelemente nur in z.B. einer oder wenigen von mehreren Stellungen des Containerdeckels relativ zur Containerwanne Verwendung finden. So können zum Beispiel bei einem quadratischen Container 1 zwei entgegen gesetzt angeordnete Verschlusslaschen 230 am Containerdeckel 200 vorgesehen sein, wobei rechts und links von jeder Verschlusslasche 230 je ein männliches Zentrierelement 120, 120A, 120B, 120C, 120D vorgesehen ist. An der Containerwanne 100 sind zwei Frontblenden 110 und zwei seitliche Blenden vorgesehen, die alle jeweils über eine Verriegelungsaufnahme 130 verfügen und wobei rechts und links jeder Verriegelungsaufnahme 130 je ein weibliches Zentrierelement 220, 220A, 220B, 220C, 220D, vorgesehen ist. Der Containerdeckel 200 ist dann immer noch in vier Stellungen auf die Containerwanne 100 aufsetzbar, jedoch werden nur von der Hälfte der weiblichen Zentrierelemente die männlichen Gegenstücke aufgenommen.

Diese und weitere Vorteile ergeben sich dem Fachmann aus dem Studium der Anmeldeunterlagen. Viele Merkmale, die hier zu den einzelnen Ausführungsbeispielen beschrieben sind, lassen sich aber auch in Kombination mit anderen Ausführungsbeispielen verwirklichen, so können z.B. auch die männlichen Zentrierelemente 120 an dem Containerdeckel vorgesehen sein und die entsprechenden weiblichen Zentrierelemente 220 können an der Containerwanne 100 vorgesehen sein. Ebenso sind die angegebenen Materialien für alle Ausführungsbeispiele anwendbar. Die Zentrierelemente können aus Metall oder Kunststoff oder beidem ausgebildet sein. Wenn die Zentrierelemente aus Kunststoff ausgebildet sind, ist es insbesondere vorteilhaft, wenn diese einen U-förmigen axialen Querschnitt aufweisen, da auf diese Weise Zentrierelemente hergestellt werden können, die durchweg im Wesentlichen dieselbe Wandstärke aufweisen. Eine einheitliche Wandstärke ist insofern Vorteilhaft, dass bei einem Spritzgussverfahren während des Abkühlvorgangs keine unebenen Oberflächen entstehen, welche häufig auftreten, wenn stark unterschiedliche Wanddicken an einem Bauteil ausgebildet werden. Vorteilhafter Weise werden die Zentrierelemente dafür von der der Stirnseite abgewandten Seite, also der für den Nutzer nicht direkt einsehbaren Seite mit Vertiefungen ausgebildet.

## Patentansprüche

1. Sterilisierbehälter (1) mit
einer Behälterwanne (100) mit einer Zugangsöffnung,
einem Behälterdeckel (200), und
einer Dichtung (201), welche im geschlossenen Zustand des Behälters (1) zwischen der Behälterwanne (100) und dem Behälterdeckel (200) angeordnet ist, wobei sich die Dichtung (201) im geschlossenen Zustand des Behälters (1) entlang des gesamten Umfangs der Zugangsöffnung mit der Behälterwanne (100) und dem Behälterdeckel (200) in Kontakt befindet, und
einer Zentrierhilfe (120, 220)
**dadurch gekennzeichnet, dass**
die Zentrierhilfe (120, 220) eine erste Zentriereinrichtung (120), die an entweder dem Behälterdeckel (200) oder der Behälterwanne (100) vorgesehen ist, und
eine zweite Zentriereinrichtung (220) aufweist, die an dem anderen von entweder dem Behälterdeckel (200) oder der Behälterwanne (100) vorgesehen ist, wobei
die Zentriereinrichtungen (120, 220) aus einem Material ausgebildet sind, das einen höheren Elastizitätsmodul als die Dichtung (201) aufweist, wobei die erste (120) und zweite (220) Zentriereinrichtung je wenigstens ein männliches und ein weibl iches Zentrierelement aufweist.

2. Behälter (1) nach Anspruch 1,
**dadurch gekennzeichnet, dass**
im geschlossenen Zustand des Behälters (1) das Spiel zwischen der ersten und zweiten Zentriereinrichtung (120, 220) geringer ist als das Spiel zwischen Behälterdeckel (200) und Behälterwanne (100) in dem Fall, in dem keine Zentriereinrichtungen (120, 220) vorgesehen sind.

3. Behälter (1) nach einem der Ansprüche 1 oder 2,
**dadurch gekennzeichnet, dass**
die erste Zentriereinrichtung (120) eine Vielzahl von männlichen Zentrierelementen (120, 120A, 120B, 120C, 120D) aufweist, und
die zweite Zentriereinrichtung (220) eine Vielzahl von weiblichen Zentrierelementen (220, 220A, 220B, 220C, 220D) aufweist.

4. Behälter (1) nach einem der Ansprüche 1 bis 3,
**dadurch gekennzeichnet, dass**
die erste und zweite Zentriereinrichtung (120, 220) jeweils wenigstens ein männliches und ein weibliches Zentrierelement (120, 120A, 120B, 120C, 120D; 220, 220A, 220B, 220C, 220D) aufweist.

5. Behälter (1) nach einem der Ansprüche 3 oder 4,
**dadurch gekennzeichnet, dass**
wenigstens ein männliches Zentrierelement (120, 120A, 120B, 120C, 120D) im Wesentlichen die Form eines Parallelepipeds - insbesondere eines Quaders - , einer Pyramide, eines Pyramidenstumpfes, eines Prismas, eines Zylinders, eines Kegels, eines Kegelstumpfes, eines Teilellipsoids oder eines aus mehreren gleichen und/oder verschiedenen der genannten Körper zusammen gesetzten Körpers aufweist, wobei das zugehörige weibliche Zentrierelement (220, 220A, 220C, 220D) vorzugsweise eine korrespondierende negative Form aufweist.

6. Behälter (1) nach einem der Ansprüche 3 bis 5,
**dadurch gekennzeichnet, dass**
wenigstens ein männliches Zentrierelement (120) und/oder ein weibliches Zentrierelement (220) einen zumindest teilweise konischen Bereich (120A1, 120B, 120C1, 120C2, 120D2; 220C, 220D) aufweist, sodass das Spiel zwischen männlichem und weiblichem Zentrierelement (120, 220) zu Beginn des Aufsetzens des Behälterdeckels (200) größer ist als im geschlossenen Zustand des Behälters (1), wobei das zugehörige weibliche Zentrierelement (220) und/oder männliche Zentrierelement (120) vorzugsweise eine korrespondierende negative bzw. positive Form aufweist.

7. Behälter (1) nach einem der vorangehenden Ansprüche,
**dadurch gekennzeichnet, dass**
die Zentriereinrichtungen (120, 220) so angeordnet sind, dass sie im geschlossenen Zustand des Behälters (1) außerhalb des Behälterinneren angeordnet sind.

8. Behälter (1) nach Anspruch 7,
**dadurch gekennzeichnet, dass**
die erste Zentriereinrichtung (120) direkt oder über je eine an den äußeren Stirnseiten der Behälterwanne (100) angeordnete Frontblende (110) an den äußeren Stirnseiten der Behälterwanne (100) vorgesehen ist, und/oder
die zweite Zentriereinrichtung (120) in der Umgebung von Verschlusselementen (230) vorgesehen ist, welche an mindestens zwei entgegen gesetzten Seiten des Behälterdeckels (200) vorgesehen sind.

9. Behälter (1) nach einem der Ansprüche 1 bis 7,
**dadurch gekennzeichnet, dass**
die erste Zentriereinrichtung (120) vier flächige Vorsprünge (120) aufweist, welche in lateraler Richtung einen Winkel gegenüber der Behälterlängsachse und der Behälterquerachse einnehmen, wobei die Vorsprünge (120) jeweils beiderseits einer Verschlussaufnahme (210) an der Oberseite von zwei Frontblenden (110) vorgesehen sind, die an beiden äußeren Stirnseiten der Behälterwanne (100) angeordnet sind, wobei
die flächigen Vorsprünge (120) an ihrer lateralen Stirnseite stärker abgeschrägt sind als an ihrer zentralen Stirnseite, sodass sich die Vorsprünge in distaler Richtung im Wesentlichen von außen her verjüngen, wobei
die zweite Zentriereinrichtung (220) vier Vertiefungen aufweist, die daran angepasst sind, die entsprechenden flächigen Vorsprünge im Wesentlichen formschlüssig aufzunehmen, wobei
jeweils zwei Vertiefungen (220) in einem Bauteil (210) ausgebildet sind, welches zwischen der inneren Stirnseite des Behälterdeckes (200) und der Dichtung (201) vorgesehen ist.

10. Behälter (1) nach einem der Ansprüche 1 bis 6,
**dadurch gekennzeichnet, dass**
die erste Zentriereinrichtung (120) an der Innenseite des Behälterdeckels (200) innerhalb der Dichtung (201) vorgesehen ist und die zweite Zentriereinrichtung (220) an der Behälterwanne (100) vorgesehen ist.

11. Behälter (1) nach einem der Ansprüche 3 bis 10,
**dadurch gekennzeichnet, dass**
die erste Zentriereinrichtung (120) aus einer geraden Anzahl von männlichen Zentrierelementen (120) besteht, die in den Ecken des Behälterdeckels (200) und/oder in der Mitte jeder Seite des Behälterdeckels (200) angeordnet sind.

12. Behälter (1) nach einem der vorangehenden Ansprüche,
**dadurch gekennzeichnet, dass**
die Zentrierhilfe (120, 220) einen punktsymmetrischen Aufbau mit einem Drehwinkel von 360°/n hat, wobei n eine positive ganze Zahl ist, vorzugsweise eine ganze Zahl größer eins.

## Claims

1. Sterilization container (1) having
a container trough (100) with an access opening,
a container lid (200), and
a seal (201) which is arranged between the container trough (100) and the container lid (200) in the closed state of the container (1), wherein the seal (201), in the closed state of the container (1), along the entire periphery of the access opening, is in contact with the container trough (100) and with the container lid (200), and
a centering aid (120, 220)
**characterized in that**
the centering aid (120, 220) has a first centering device (120), which is provided on either the container lid (200) or the container trough (100), and
a second centering device (220), which is provided on the other one of either the container lid (200) or the container trough (100), wherein
the centering devices (120, 220) are made from a material which has a higher modulus of elasticity than the seal (201),
wherein the first (120) and second (220) centering devices each have at least one male and one female centering element.

2. Container (1) according to claim 1,
**characterized in that,**
in the closed state of the container (1), the clearance between the first and second centering devices (120, 220) is smaller than the clearance between the container lid (200) and the container trough (100) in the event that no centering devices (120, 220) are provided.

3. Container (1) according to one of claims 1 or 2,
**characterized in that**
the first centering device (120) has a plurality of male centering elements (120, 120A, 120B, 120C, 120D), and
the second centering device (220) has a plurality of female centering elements (220, 220A, 220B, 220C, 220D).

4. Container (1) according to one of claims 1 through 3,
**characterized in that**
the first and second centering devices (120, 220) each have at least one male and one female centering element (120, 120A, 120B, 120C, 120D; 220, 220A, 220B, 220C, 220D).

5. Container (1) according to one of claims 3 or 4,
**characterized in that**
at least one male centering element (120, 120A, 120B, 120C, 120D) essentially has the shape of a parallelepiped - in particular, a cuboid - a pyramid, a truncated pyramid, a cone, a truncated cone, a partial ellipsoid, or a body composed of one of several similar and/or different examples of the bodies mentioned, wherein the associated female centering element (220, 220A, 220C, 220D) preferably has a corresponding negative shape.

6. Container (1) according to one of claims 3 through 5,
**characterized in that**
at least one male centering element (120) and/or a female centering element (220) has an at least partially conical area (120A1, 120B, 120C1, 120C2, 120D2; 220C, 220D), so that the clearance between the male and the female centering elements (120, 220) at the beginning of the placement of the container lid (200) is greater than in the closed state of the container (1), wherein the associated female centering element (220) and/or male centering element (120) preferably have corresponding negative and/or positive shapes.

7. Container (1) according to one of the previous claims,
**characterized in that**
the centering devices (120, 220) are arranged such that, in the closed state of the container (1), they are arranged outside the interior of the container (1).

8. Container (1) according to claim 7,
**characterized in that**
the first centering device (120) is provided directly on the external front faces of the container trough (100), or via a front panel (110) arranged on the external front faces of the container trough (100), and/or
the second centering device (120) is provided in the surroundings of fastening elements (230) which are provided on at least two opposite sides of the container lid (200).

9. Container (1) according to one of claims 1 through 7,
**characterized in that**
the first centering device (120) has four planar projections (120) which run in a lateral direction to form an angle in relation to the longitudinal axis of the container and the transverse axis of the container, wherein the projections (120) are provided on both sides of a fastening mount (210) on the top of two front panels (110) which are arranged on both outer front faces of the container trough (100), wherein
the planar projections (120) are more strongly beveled on their lateral front faces than on their central front faces, so that the projections essentially taper in a distal direction from the outside, wherein
the second centering device (220) has four recesses which are adjusted so as to accommodate the corresponding planar projections in, essentially, a positive fit, wherein
two recesses (220) are each formed in a component (210) which is provided between the internal front face of the container lid (200) and the seal (201).

10. Container (1) according to one of claims 1 through 6,
**characterized in that**
the first centering device (120) is provided on the inside of the container lid (200) within the seal (201), and the second centering device (220) is provided on the container trough (100).

11. Container (1) according to one of claims 3 through 10,
**characterized in that**
the first centering device (120) consists of an even number of male centering elements (120) which are arranged in the corners of the container lid (200) and/or in the middle of each side of the container lid (200).

12. Container (1) according to one of the previous claims,
**characterized in that**
the centering aid (120, 220) has a point-symmetrical construction with an angle of rotation of 360°/n, wherein n is a positive integer - preferably, an integer greater than one.

## Revendications

1. Récipient de stérilisation (1) présentant
une cuve (100) de récipient pourvue d'un orifice d'accès,
un couvercle (200) de récipient et
un joint (201), qui est disposé, dans l'état fermé du récipient (1), entre la cuve (100) de récipient et le couvercle (200) de récipient, le joint (201) se trouvant, dans l'état fermé du récipient (1), le long de la périphérie totale de l'ouverture d'accès, en contact avec la cuve (100) de récipient et le couvercle (200) de récipient et
un auxiliaire de centrage (120, 220)
**caractérisé en ce que**
l'auxiliaire de centrage (120, 220) présente un premier dispositif de centrage (120), qui est disposé soit au niveau du couvercle (200) de récipient, soit au niveau de la cuve (100) de récipient et
un deuxième dispositif de centrage (220), qui est disposé au niveau de l'autre parmi le couvercle (200) de récipient ou la cuve (100) de récipient,
les dispositifs de centrage (120, 220) étant réalisés en un matériau qui présente un module d'élasticité supérieur à celui du joint (201),
le premier (120) et le deuxième (220) dispositif de centrage présentant respectivement au moins un élément de centrage mâle et un élément de centrage femelle.

2. Récipient (1) selon la revendication 1,
**caractérisé en ce que,**
dans l'état fermé du récipient (1), le jeu entre le premier et le deuxième dispositif de centrage (120, 220) est plus petit que le jeu entre le couvercle (200) de récipient et la cuve (100) de récipient dans le cas où aucun dispositif de centrage (120, 220) n'est prévu.

3. Récipient (1) selon l'une des revendications 1 ou 2,
**caractérisé en ce que**
le premier dispositif de centrage (120) présente une pluralité d'éléments de centrage mâles (120, 120A, 120B, 120C, 120D) et
le deuxième dispositif de centrage (220) présente une pluralité d'éléments de centrage femelles (220, 220A, 220B, 220C, 220D).

4. Récipient (1) selon l'une des revendications 1 à 3,
**caractérisé en ce que**
les premier et deuxième dispositifs de centrage (120, 220) présentent respectivement au moins un élément de centrage mâle et un femelle (120, 120A, 120B, 120C, 120D ; 220, 220A, 220B, 220C, 220D).

5. Récipient (1) selon l'une des revendications 3 ou 4,
**caractérisé en ce que**
au moins un élément de centrage mâle (120, 120A, 120B, 120C, 120D) présente, essentiellement, la forme d'un parallélépipède - en particulier d'un parallélépipède rectangle -, d'une pyramide, d'une pyramide tronquée, d'un prisme, d'un cylindre, d'un cône, d'un cône tronqué, d'un ellipsoïde partiel ou d'un corps composé d'un ou de plusieurs des corps mentionnés, identiques et/ou différents, l'élément de centrage femelle (220, 220A, 220C, 220D) associé présentant de préférence une forme négative correspondante.

6. Récipient (1) selon l'une des revendications 3 à 5,
**caractérisé en ce que**
au moins un élément de centrage mâle (120) et/ou un élément de centrage femelle (220) présente(nt) une zone (120A1, 120B, 120C1, 120C2, 120D2 ; 220C, 220D) au moins partiellement conique, de manière telle que le jeu entre l'élément de centrage mâle et femelle (120, 220), au début de la mise en place du couvercle (200) de récipient, est supérieur à celui dans l'état fermé du récipient (1), l'élément de centrage femelle (220) associé et/ou l'élément de centrage mâle (120) associé présentant de préférence une forme négative ou, selon le cas, positive correspondante.

7. Récipient (1) selon l'une des revendications précédentes,
**caractérisé en ce que**
les dispositifs de centrage (120, 220) sont disposés de manière telle qu'ils sont disposés, dans l'état fermé du récipient (1), en dehors de l'intérieur du récipient.

8. Récipient (1) selon la revendication 7,
**caractérisé en ce que**
le premier dispositif de centrage (120) se situe, directement ou au-dessus d'une tôle avant (110) respective disposée au niveau des faces frontales externes de la cuve (100) de récipient, au niveau des faces frontales externes de la cuve (100) de récipient et/ou
le deuxième dispositif de centrage (120) se situe dans l'environnement d'éléments de verrouillage (230), qui sont situés au niveau d'au moins deux faces opposées du couvercle (200) de récipient.

9. Récipient (1) selon l'une des revendications 1 à 7,
**caractérisé en ce que**
le premier dispositif de centrage (120) présente quatre parties en saillie (120) plates qui forment, dans la direction latérale, un angle par rapport à l'axe du récipient et à l'axe transversal du récipient, les parties en saillie (120) étant situées à chaque fois des deux côtés d'un évidement de verrouillage (210) au niveau du côté supérieur de deux tôles avant (110) qui sont disposées au niveau des deux faces frontales externes de la cuve (100) de récipient,
les parties en saillie (120) plates étant plus en biais au niveau de leur face frontale latérale qu'au niveau de leur face frontale centrale, de telle sorte que les parties en saillie se rétrécissent essentiellement à partir de l'extérieur dans la direction distale,
le deuxième dispositif de centrage (220) présentant quatre creux qui sont conçus pour recevoir les parties en saillie plates correspondantes essentiellement par complémentarité de forme,
deux creux (220) étant à chaque fois formés dans une pièce (210), qui est située entre la face frontale interne du couvercle (200) de récipient et le joint (201).

10. Récipient (1) selon l'une des revendications 1 à 6,
**caractérisé en ce que**
le premier dispositif de centrage (120) est situé au niveau du côté interne du couvercle (200) de récipient dans le joint (201) et le deuxième dispositif de centrage (220) est situé au niveau de la cuve (100) de récipient.

11. Récipient (1) selon l'une des revendications 3 à 10,
**caractérisé en ce que**
le premier dispositif de centrage (120) est constitué par un nombre pair d'éléments de centrage (120) mâles, qui sont disposés dans les coins du couvercle (200) de récipient et/ou au milieu de chaque côté du couvercle (200) de récipient.

12. Récipient (1) selon l'une des revendications précédentes,
**caractérisé en ce que**
l'auxiliaire de centrage (120, 220) présente une construction dotée d'une symétrie ponctuelle avec un angle de rotation de 360°/n, n étant un nombre entier positif, de préférence un nombre entier supérieur à un.
